# EUROPÄISCHE PATENTSCHRIFT

(11) **EP 1 736 774 B1**
(45) Veröffentlichungstag und Bekanntmachung des Hinweises auf die Patenterteilung: **06.02.2008**
(21) Anmeldenummer: 05013452.7
(22) Anmeldetag: 22.06.2005
(51) Int. Cl.: G01N 33/50, G01N 21/55, G02B 1/04

(54) **Analysesystem zur Analyse einer Probe auf einem analytischen Testelement**
Analytic system for the analysis of a sample on an analytic test element
Système analytique pour l'analyse d'un échantillon sur un élément analytique

(43) Veröffentlichungstag der Anmeldung: 27.12.2006
(73) Patentinhaber: F.HOFFMANN-LA ROCHE AG, 4070 Basel (CH); Roche Diagnostics GmbH, 68305 Mannheim (DE)
(72) Erfinder: Steeg, Klaus-Dieter, 76709 Kronau (DE); Schulat, Jochen, 68167 Mannheim (DE)
(74) Vertreter: Isenbruck, Günter

(56) Entgegenhaltungen:
- DE-A1- 2 637 514
- DE-A1- 19 844 500
- [Online] XP002363465 Produktinformation "Akku-Chek Compact" von ROCHE DIAGNOSTICS Gefunden im Internet: URL:http://www.accu-chek.de/monitoring/de/ content/accu_chek_produkte/ systeme/accu-chek_compact.html> [gefunden am 2006-01-18]

## Beschreibung

Die vorliegende Erfindung betrifft ein Analysesystem zur Analyse einer Probe auf einem analytischen Testelement mit einem eine Linse und eine Blende umfassenden Optikmodul.

Zur Analyse von Proben, beispielsweise Körperflüssigkeiten wie Blut oder Urin, werden häufig Analysesysteme verwendet, bei denen sich die zu analysierenden Proben auf einem Testelement befinden und in einem Testfeld gegebenenfalls mit einer oder mehreren Reagenzien auf dem Testelement reagieren, bevor sie analysiert werden. Die optische, insbesondere photometrische, und die elektrochemische Auswertung von Testelementen stellen die gebräuchlichsten Verfahren zur schnellen Bestimmung der Konzentration von Analyten in Proben dar. Analysesysteme mit Testelementen zur Probenanalyse werden allgemein im Bereich der Analytik, der Umweltanalytik und vor allem im Bereich der medizinischen Diagnostik eingesetzt. Insbesondere im Bereich der Blutglukosediagnostik aus Kapillarblut besitzen Testelemente, die photometrisch oder elektrochemisch ausgewertet werden, einen großen Stellenwert.

Es gibt verschiedene Formen von Testelementen. Bekannt sind zum Beispiel im Wesentlichen quadratische Plättchen, die auch als Slides bezeichnet werden, in deren Mitte sich ein mehrschichtiges Testfeld befindet. Diagnostische Testelemente, die streifenförmig ausgebildet sind, werden als Teststreifen bezeichnet. Im Stand der Technik sind Testelemente umfassend beschrieben, beispielsweise in den Dokumenten DE-A 197 53 847, EP-A 0 821 233, EP-A 0 821 234 oder WO 97/02487. Die vorliegende Erfindung bezieht sich auf Testelemente beliebiger Form, insbesondere auf streifenförmige Testelemente.

Zur analytischen Untersuchung einer Probe auf einem Testelement sind im Stand der Technik Testelement-Analysesysteme bekannt, die eine Testelement-Aufnahme zum Positionieren des Testelements in einer Messposition und eine Mess- und Auswerteeinrichtung zur Durchführung einer Messung und Ermittlung eines hierauf resultierenden Analyseresultats enthalten.

WO 00/19185 A1 bezieht sich auf eine Vorrichtung zur photometrischen Auswertung von Testelementen, beinhaltend
- eine Beleuchtungseinheit mit mindestens einer ersten und einer zweiten Lichtquelle,
- eine Halterung zur Aufnahme eines Testelements mit einer Nachweiszone in der Weise, dass die Nachweiszone gegenüber der Beleuchtungseinheit positioniert wird,
- eine Detektionseinheit mit mindestens einem Detektor, der von der Nachweiszone reflektiertes oder durch die Nachweiszone transmittiertes Licht detektiert,
- eine Steuerungseinheit, die die beiden Lichtquellen aktiviert und das von der Detektionseinheit generierte Signal als Detektionssignal aufnimmt und
- eine Auswerteeinheit, die die Detektionssignale auswertet, um die in der Probe enthaltene Analytkonzentration zu ermitteln.

EP 0 618 443 A1 bezieht sich auf ein Teststreifen-Analysesystem bestehend aus einem Auswertegerät mit einer Teststreifenhalterung und den passenden Teststreifen. Die Teststreifenhalterung dient dazu, den Teststreifen in einer definierten Position gegenüber einer Messeinheit zu positionieren. Sie weist eine Teststreifen-Auflage und eine Führung für den Teststreifen auf.

WO 01/48461 A1 hat ein Testelement-Analysesystem zur analytischen Untersuchung einer Probe zum Gegenstand. Das Analysesystem umfasst Testelemente mit einer Tragfolie und einem an einer Flachseite der Tragfolie befestigten Testfeld, das zur Durchführung einer Analyse derartig mit der Probe in Kontakt gebracht wird, dass flüssige Probenbestandteile in das Testfeld eindringen, wobei das Testfeld ein Reagenzsystem enthält, dessen Reaktion mit Bestandteilen der Probe zu einer optisch messbaren, für die Analyse charakteristischen Änderung in einer Detektionszone und auf der der Tragfolie zugewandten Seite des Testfeldes führt. Ferner umfasst das Analysesystem ein Auswertegerät mit einer Testelementhalterung zur Positionierung eines Testelements in einer Messposition und eine Messeinrichtung zur Messung der optisch messbaren Änderung in der Detektionszone, wobei die Messeinrichtung einen Lichtsender zur Beleuchtung der Detektionszone mit Primärlicht und einen Detektor zur Detektion des dabei von der Detektionszone diffus reflektierten Sekundärlichtes aufweist.

Viele solcher bekannter Analysesysteme weisen mindestens ein Optikmodul auf, das unter anderem eine Linse und eine Blende umfasst, durch die Licht fokussiert werden kann. Diese Optikmodule werden im Stand der Technik aus mehreren Einzelteilen hergestellt, die zusammengesetzt und zum Beispiel mittels Ultraschallschweißen, Warmverstemmen oder Verkleben miteinander verbunden werden. Dabei müssen die Linse und die Blendenöffnung räumlich genau zueinander entsprechend dem Strahlengang des Lichts positioniert werden. Das Zusammenfügen der Teile ist nur mit großem Aufwand durch ihre Toleranzen und geringe Größe möglich. Durch viele Einzelteile entsteht außerdem eine große Gesamttoleranz des Optikmoduls.

Aufgabe der vorliegenden Erfindung ist es daher, die Nachteile des Standes der Technik zu vermeiden. Insbesondere sollen der Aufwand und die Kosten bei dem Zusammenbau des Analysesystems reduziert werden.

Diese Aufgabe wird erfindungsgemäß gelöst durch ein Analysesystem zur Analyse einer Probe auf einem analytischen Testelement, umfassend
- ein Messmodul zur Durchführung von Messungen an der Probe auf dem analytischen Testelement und
- ein Optikmodul, das eine Linse und eine Blende umfasst, durch die Licht fokussiert werden kann, wobei
- die Linse und die Blende des Optikmoduls einstückig in einem MehrkomponentenSpritzgießteil zusammengefasst sind.

Das erfindungsgemäße Analysesystem enthält unter anderem ein Messmodul zur Durchführung von Messungen an einer Probe auf einem analytischen Testelement.

Bei der Probe handelt es sich zum Beispiel um eine Körperflüssigkeit, insbesondere um Blut oder interstitielle Flüssigkeit. Die Untersuchung von Blutproben oder von interstitieller Flüssigkeit ermöglichen in der klinischen Diagnostik das frühzeitige und zuverlässige Erkennen von pathologischen Zuständen sowie die gezielte und fundierte Kontrolle von Körperzuständen. Die medizinische Diagnostik setzt stets die Gewinnung einer Probe aus Blut oder interstitieller Flüssigkeit des zu untersuchenden Individuums voraus.

Zur Gewinnung der Probe kann die Haut zum Beispiel an der Fingerbeere oder dem Ohrläppchen der zu untersuchenden Person mit Hilfe einer sterilen, scharfen Lanzette perforiert werden, um so eine geringe Menge Blut oder interstitielle Flüssigkeit für die Analyse zu gewinnen. Insbesondere eignet sich diese Methode für die Analyse einer Probe, die unmittelbar nach der Probengewinnung durchgeführt wird.

Vor allem im Bereich des sogenannten "home-monitoring", also dort, wo medizinische Laien selbst einfache Analysen des Blutes oder der interstitiellen Flüssigkeit durchführen, und dort insbesondere für die regelmäßige, mehrmals täglich durchzuführende Blutgewinnung durch Diabetiker für die Kontrolle der Blutglukosekonzentration, werden Lanzetten und dazu passende Geräte (sogenannte Stechhilfen) angeboten, die eine möglichst schmerzarme und reproduzierbare Probengewinnung ermöglichen.

Die Probe wird zur Durchführung der Messungen auf ein analytisches Testelement aufgebracht, das Reagenzien (zum Beispiel in einem Testfeld) enthält. Wenn die Reagenzien die Probe kontaktieren, führt eine Reaktion des in der Probe enthaltenen Analyten mit den Reagenzien zu einer physikalisch messbaren Veränderung des Testelements, die mit der Konzentration des Analyten korreliert.

Das Messmodul des erfindungsgemäßen Analysesystems dient zum Messen dieser Veränderung. Die bei den Messungen des Messmoduls erhaltenen Messwerte dienen zur Bestimmung der Konzentration des Analyten in der Probe.

Bei photometrischen Analysesystemen enthalten die Testelemente ein Reagenzsystem, dessen Reaktion mit dem Analyten zu einer photometrisch nachweisbaren Veränderung (einem Farbumschlag) führt. Die Reagenzien befinden sich dabei üblicherweise in einem Testfeld des Testelements, wobei deren Farbe sich in Abhängigkeit von der Konzentration ändert. Diese Farbänderung lässt sich mit Hilfe eines Messmoduls quantitativ durch Reflexionsphotometrie bestimmen.

Elektrochemische Testelemente enthalten ein elektrochemisches Reagenzsystem, dessen Reaktion mit dem Analyten die zwischen zwei Polen des Testelements anliegende elektrische Spannung und/oder die zwischen zwei Polen des Testelements bei definierter Spannung fließende Stromstärke beeinflusst. In diesem Fall ist also die Spannung oder Stromstärke die physikalisch messbare Größe, die mit einer entsprechenden in dem Analysesystem integrierten, als Spannungs- oder Strommesseinrichtung gestalteten Messmodul bestimmt und deren mit der Konzentration des Analyten korrelierende Änderung in die Analysedaten (Konzentration des Analyten) umgerechnet wird.

Ein Optikmodul im Sinne der vorliegenden Erfindung ist eine Baugruppe, die unter anderem mindestens eine Linse und eine Blende enthält. Durch die Linse und die Blende kann Licht fokussiert werden. Als Linse wird in diesem Zusammenhang ein dem Fachmann als optische Linse bekanntes optisches Bauteil bezeichnet. Eine Blende bezeichnet in diesem Zusammenhang ein optisches Bauteil, das einen lichtundurchlässigen Blendenkörper und eine lichtdurchlässige Blendenöffnung umfasst. Eine Blende verhindert die Ausbreitung von Licht in bestimmten Raumrichtungen. Sie dient zur Begrenzung des Querschnittes von Strahlenbündeln und der Verringerung von Streulicht.

In dem erfindungsgemäßen Analysesystem sind die Linse und die Blende des Optikmoduls einstückig in einem Mehrkomponentenspritzgießbauteil zusammengefasst. Spritzgießen ist ein im Stand der Technik bekanntes Verfahren, bei dem ein plastifizierter Werkstoff (Spritzgießmasse) (insbesondere ein Thermo- oder Duroplaste) in ein Umformwerkzeug (Spritzgießwerkzeug) mit hohem Druck eingespritzt wird und dort unter Druckeinwirkung in den festen Zustand übergeht. Das Spritzgießteil kann dann dem Spritzgießwerkzeug entnommen werden. Mehrkomponentenspritzgießen ist ebenfalls ein im Stand der Technik bekanntes Verfahren. Insbesondere eignet sich das sogenannte Verbundspritzgießen zur Herstellung des Mehrkomponentenspritzgießbauteils für das erfindungsgemäße Analysegerät. Dabei werden zwei oder mehr Materialien nacheinander in ein Spritzgießwerkzeug gespritzt, wodurch sie an ihren Grenzflächen materialschlüssig zusammengefügt werden. Die Geometrie der in dem Spritzgießwerkzeug vorhandenen Kavität wird zwischen den verschiedenen Einspritzungen verändert.

Im Stand der Technik werden zum Beispiel mehrere Linsen als einstückiges Mehrkomponentenspritzgießbauteil hergestellt, wie es zum Beispiel in DE 102 61 974 A1, US 2004/0120053 A1 oder DE 44 31 744 A1 beschrieben ist.

Bei dem erfindungsgemäßen Analysesystem ergibt sich durch das einstückige Zusammenfassen der Linse und der Blende des Optikmoduls in dem Mehrkomponentenspritzgießbauteil eine Vielzahl von Vorteilen. Ein Zusammenfügen von Linse und Blende nach ihrer Herstellung ist nicht mehr notwendig, wodurch ein Arbeitsschritt bei der Herstellung des erfindungsgemäßen Analysesystems entfällt. Daraus ergeben sich unter anderem Kosteneinsparungen. Die Handhabung des Mehrkomponentenspritzgießbauteils ist einfacher als die der einzelnen Linse und der einzelnen Blende. Es lässt sich eine wiederholbar genaue Einheit aus Linse und Blende auch in der Serienfertigung produzieren. Zwischen Linse und Blende bestehen keine Toleranzen. So wird eine exakte Positionierung der Blende relativ zu der Linse gewährleistet.

In dem Mehrkomponentenspritzgießteil des Optikmoduls können bei dem erfindungsgemäßen Analysesystem neben der Linse und der Blende auch weitere Linsen und/oder weitere Blenden und/oder weitere Bestandteile des Optikmoduls zusammengefasst sein. Gegebenenfalls ist das gesamte Optikmodul ein Mehrkomponentenspritzgießteil.

Gemäß einer bevorzugten Ausführungsform der vorliegenden Erfindung sind in dem Mehrkomponentenspritzgießteil zwei Blenden und eine oder zwei Linsen zusammengefasst. Vorzugsweise ist das Mehrkomponentenspritzgießteil ein Zweikomponentenspritzgießteil, insbesondere ein Zweikomponentenspritzgießteil mit einer ersten lichtdurchlässigen Kunststoffkomponente und einer zweiten lichtundurchlässigen Kunststoffkomponente. Die lichtdurchlässige Kunststoffkomponente ist bevorzugt durchlässig für Licht in einem Wellenlängenbereich von 200 bis 1700 nm, besonders bevorzugt in einem Wellenlängenbereich von 600 bis 950 nm, wobei die lichtundurchlässige Kunststoffkomponente vorzugsweise für Licht in diesem Wellenlängenbereich weitgehend nicht durchlässig ist. Vorzugsweise besteht die in das Mehrkomponentenspritzgießteil integrierte Linse aus dem lichtdurchlässigen Kunststoff. Dazu ist ein Bereich des Mehrkomponentenspritzgießteils aus dem lichtdurchlässigen Kunststoff so geformt, dass er die Funktion einer optischen Linse übernimmt. Ein lichtundurchlässiger Kunststoff kann zum Beispiel für Bereiche des Mehrkomponentenspritzgießteils verwendet werden, die die Funktion eines Blendenkörpers übernehmen. Die lichtdurchlässige Kunststoffkomponente kann zum Beispiel mindestens einen Kunststoff ausgewählt aus der Gruppe Acrylnitril-Butadien-Styrol-Polymerisate (ABS), Methylmethacrylat-Acrylnitril-Butadien-Styrol-Copolymere (MABS), Polycarbonat (PC), Polycarbonat-Blends (PCB), Polysulfon (PSU), Polyethersulfon (PES) enthalten. Die lichtundurchlässige Kunststoffkomponente enthält vorzugsweise mindestens einen Kunststoff ausgewählt aus der Gruppe Acrylnitril-Butadien-Styrol-Polymerisate (ABS), Polycarbonat-Blends (PCB) und Polyethersulfon (PES).

Gemäß einer bevorzugten Ausführungsform der vorliegenden Erfindung umfasst die Blende einen Blendenkörper aus einem lichtundurchlässigen Kunststoff und eine Blendenöffnung, wobei die Blendenöffnung mit einem lichtdurchlässigen Kunststoff gefüllt ist. Die Blendenöffnung ist also mit einem schützenden Fenster aus dem lichtdurchlässigen Kunststoff verschlossen, das Verschmutzungen über die Blendenöffnung verhindert. Besonders bevorzugt werden dabei die Linse und die mit dem lichtdurchlässigen Kunststoff gefüllte Blendenöffnung in einem zusammenhängenden Bereich zusammengefasst. Sie sind hierbei aus dem lichtdurchlässigen Kunststoff und werden in dem Mehrkomponentenspritzgießteil zusammengefasst. Dadurch vereinfachen sich das Spritzgießverfahren und das Optikmodul und es wird eine fest definierte Positionierung der Blendenöffnung (beziehungsweise des Fensters) relativ zu der Linse gewährleistet.

Die Blende in dem Optikmodul des erfindungsgemäßen Analysesystems kann aber auch einen Blendenkörper aus einem lichtundurchlässigen Kunststoff und eine Blendenöffnung umfassen, wobei die Blendenöffnung eine Aussparung in dem Blendenkörper ist. Die Aussparung ist dabei nicht mit Material gefüllt. Dadurch wird weniger Licht beim Durchqueren der Blendenöffnung absorbiert, als wenn die Blendenöffnung mit einem Material gefüllt ist.

Gemäß einer bevorzugten Ausführungsform der vorliegenden Erfindung enthält das Messmodul das Optikmodul, wobei das Optikmodul zur Durchführung von optischen Messungen an der Probe auf dem analytischen Testelement dient. Es handelt sich dabei insbesondere um ein für photometrische Messungen an der Probe (zum Beispiel einer Körperflüssigkeit von Menschen oder Tieren) vorgesehenes Messmodul, das zur Bestimmung der Konzentration eines Analyten (zum Beispiel Glukose) in der Probe ausgelegt ist.

Im Stand der Technik wird beispielsweise in dem AKKU CHEK^{®}-Compakt Analysesystem von Roche Diagnostics, Deutschland derzeit ein Messmodul eingesetzt, das ein Optikmodul enthält, wobei das Optikmodul aus zwei Kunststoffteilen zusammengesetzt wird, wovon das eine eine Blende mit Kunststofffenster als Blendenöffnung und das andere eine Linse enthält. Die Teile haben eine geringe Größe (14,5 x 7,5 x 21 mm beziehungsweise 0,7 x 4 x 6 mm). Die Aufgabe des Messmoduls in diesem Analysesystem aus dem Stand der Technik ist es, ein Testelement für die Durchführung von Messungen zu positionieren und mit Hilfe des Optikmoduls Lichtstrahlen darauf zu richten, um optisch Blutzuckerwerte zu bestimmen. Die zwei Kunststoffteile des Optikmoduls werden bei diesem Analysesystem mittels Ultraschallverschweißung miteinander verbunden. Dabei entstehen durch das Zusammenfügen der beiden Teile hohe Kosten. Durch die kleine Baugröße der Teile ist die Handhabung sehr kompliziert. Das Verschweißen erfordert einen zusätzlichen Arbeitsgang, der mit großem Aufwand durchgeführt wird. Zusätzliche Toleranzen durch zwei Teile können nicht vermieden werden. Die Position der Blende wird hier auch durch ihre Herstellungstoleranzen bestimmt.

In dem erfindungsgemäßen Analysesystem enthält das Messmodul ein Optikmodul, in dem mindestens eine Linse und mindestens eine Blende einstückig in einem Mehrkomponentenspritzgießteil zusammengefasst sind. Besonders bevorzugt ist, dass das in dem Messmodul enthaltene gesamte Optikmodul ein Zweikomponentenspritzgießteil ist, in dem mindestens eine Linse und mindestens eine Blende enthalten sind. Ein großer Vorteil liegt dabei darin, dass das komplette Optikmodul in einem Vorgang gespritzt werden kann. Dazu muss der optische Strahlengang gegebenenfalls neu berechnet und es müssen gegebenenfalls die in dem Optikmodul enthaltenen optischen Bauteile anders ausgelegt werden.

Vorzugsweise umfasst das Messmodul in dem erfindungsgemäßen Analysesystem eine Lichtquelle, einen Detektor und eine Testelementaufnahme, die so angeordnet sind, dass Licht von der Lichtquelle durch einen lichtdurchlässigen Bereich des Mehrkomponentenspritzgießteils zu einem in der Testelementaufnahme angeordneten Testelement und reflektiert von dem Testelement durch den lichtdurchlässigen Bereich zu dem Detektor gelangen kann. Die Lichtquelle ist zum Beispiel eine Licht emittierende Diode (LED). Der Detektor ist zum Beispiel eine Photodiode. Die Testelementaufnahme dient zur Aufnahme eines Testelements, insbesondere während der Durchführung der Messungen mit dem Optikmodul. Sie ist zur Führung beim manuellen oder automatischen Einlegen des Testelements in das Messmodul und zur genauen Positionierung des Testelements während der Messungen ausgelegt. Während der Messungen ist das Testelement in der Testelementaufnahme so positioniert, dass das Licht von der Lichtquelle über den lichtdurchlässigen Bereich des Mehrkomponentenspritzgießteils auf ein die Probe und Reagenzien enthaltendes Testfeld auf dem Testelement gerichtet wird. Je nach Konzentration des Analyten in der Probe wird ein Anteil des auf das Testelement treffenden Lichts an diesem so reflektiert, dass es durch den lichtdurchlässigen Bereich des Mehrkomponentenspritzgießteils zu dem Detektor gelangt.

Gemäß einer anderen Ausführungsform der vorliegenden Erfindung umfasst das Messmodul eine Lichtquelle, einen Detektor und eine Testelementaufnahme, die so angeordnet sind, dass Licht von der Lichtquelle durch einen lichtdurchlässigen Bereich des Mehrkomponentenspritzgießteils zu einem in der Testelementaufnahme angeordneten Testelement und transmittiert durch dieses zu dem Detektor gelangen kann.

Gemäß einer weiteren bevorzugten Ausführungsform der vorliegenden Erfindung enthält das Analysesystem ein Lesemodul zum Lesen optisch codierter Daten, wobei das Lesemodul das oder ein weiteres Optikmodul enthält.

Im Stand der Technik sind Analysesysteme bekannt, die ein Vorratsbehältnis (Magazin) mit einer Vielzahl von Testelementen enthalten. Dabei wird ein Testelement zum Beispiel mit einem Schieber oder Stößel aus dem Magazin an den Ort der Messung im Messmodul transportiert und nach der Durchführung der Messung automatisch aus dem Analysesystem ausgeworfen oder in dem Magazin remagaziniert. Beispielsweise ist aus DE 199 02 601 A1 eine Vorrichtung zum Entnehmen eines analytischen Verbrauchsmittels, insbesondere eines Testelements, aus einem Vorratsbehältnis bekannt, das eine oder mehrere Kammern aufweist, die die Verbrauchsmittel enthalten. Die Kammern weisen jeweils eine Entnahmeöffnung zum Entnehmen eines Verbrauchsmittels und eine der Entnahmeöffnung gegenüberliegende Einschuböffnung zum Einführen eines Stößels für den Transport des Verbrauchsmittels auf. Die Entnahmeöffnung und die Einschuböffnung sind zur Lagerung des Verbrauchsmittels mit einer Folie verschlossen. Die Vorrichtung umfasst einen Stößel, der zur Entnahme eines Verbrauchsmittels mittels einer Antriebseinheit verfahrbar ist.

In dem Analysesystem AKKU CHEK^{®}-Compact von Roche Diagnostics, Deutschland ist zum Beispiel ein Lesemodul enthalten (mit einem Barcode-Reader), das einen Barcode auf der äußeren Fläche eines in das Analysesystem eingelegten trommelförmigen Testelement-Magazins lesen kann. Der Barcode enthält zum Beispiel Informationen über die in dem Magazin enthaltenen Testelemente, die für die Auswertung der von dem Messmodul gemessenen Daten relevant sind und dabei berücksichtigt werden. Im Stand der Technik enthält das Lesemodul zwei einzelne Kunststoffspritzgießteile und eine Platine, die bei der Herstellung des Lesemoduls zusammengefügt werden. Das eine Spritzgießteil enthält eine Blende und das andere enthält eine Linsenanordnung. Die Platine und die zwei Spritzgießteile werden in einem komplizierten Klebevorgang miteinander verbunden. Das Zusammenfügen ist dabei nur mit großem Aufwand unter anderem aufgrund der Toleranzen der Spritzgießteile möglich. Die Handhabung ist wegen der geringen Größe der Teile problematisch. Durch die vielen Einzelteile entsteht eine große Gesamttoleranz des Optikmoduls.

Die beiden Spritzgießteile werden in einer bevorzugten Ausführungsform der vorliegenden Erfindung einstückig in einem Mehrkomponentenspritzgießteil zusammengefasst. Somit sind Blende und Linsenanordnung positionsgenau zueinander herstellbar und die genannten Nachteile können vermieden werden.

Es ist kein Klebevorgang zwischen der Blende und der Optik notwendig. Wiederholbar genaue Teile werden auch in der Serienfertigung erhalten. Die Handhabung des Mehrkomponentenspritzgießteils ist einfacher als die Handhabung der zwei Einzelteile. Es bestehen keine Toleranzen zwischen Optik und Blende. Der Aufbau ist preiswerter, so dass eine Kostenersparung erreicht wird.

Die Platine des Lesemoduls kann nachträglich an dem Mehrkomponentenspritzgießteil befestigt werden. Die Platine trägt zum Beispiel einen Detektor (beispielsweise eine Photodiode) und eine Lichtquelle (beispielsweise eine LED).

Gemäß einer bevorzugten Erfindung der vorliegenden Erfindung umfasst das Lesemodul eine Lichtquelle, einen Detektor und eine Magazinaufnahme, die so angeordnet sind, dass Licht von der Lichtquelle durch einen lichtdurchlässigen Bereich des Mehrkomponentenspritzgießteils zu einem in der Magazinaufnahme aufgenommenen Testelement-Magazin und reflektiert von dem Testelement-Magazin durch den lichtdurchlässigen Bereich zu dem Detektor gelangen kann.

Die Platine, die vorzugsweise den Detektor und die Lichtquelle trägt, wird vorzugsweise mit Hilfe von in Ausnehmungen eingreifenden Stiften relativ zu dem Mehrkomponentenspritzgießteil positioniert und dann mit diesem zum Beispiel durch Verkleben, Ultraschallverschweißen oder Warmverstemmen verbunden. Dabei weist das Mehrkomponentenspritzgießteil Ausnehmungen und/oder Stifte auf und die Platine dazu passende Stifte und/oder Ausnehmungen.

Die Erfindung bezieht sich weiterhin auf ein Verfahren zur Herstellung eines Analysesystems zur Analyse einer Probe auf einem analytischen Testelement, wobei das Analysesystem ein Messmodul und ein Optikmodul enthält, wobei das Optikmodul eine Linse und eine Blende umfasst, gekennzeichnet durch folgende Schritte:
- Mehrkomponentenspritzgießen eines einstückigen Mehrkomponentenspritzgießteils, in dem die Linse und die Blende zusammengefasst sind und
- Positionieren und Montieren des Mehrkomponentenspritzgießteils in dem Analysesystem.

Anhand der Zeichnung wird die Erfindung nachstehend näher erläutert.

### Es zeigt:

- Figur 1: einen Schnitt durch ein Optikmodul, das in einem Messmodul eines Analysesystems aus dem Stand der Technik enthalten ist,
- Figur 2: einen Schnitt durch ein Optikmodul, das in einem Messmodul eines erfindungsgemäßen Analysesystems enthalten ist,
- Figur 3: einen Schnitt durch ein Messmodul eines erfindungsgemäßen Analysesystems mit Testelement,
- Figur 4: den Strahlengang in dem Messmodul gemäß Figur 3,
- Figur 5: schematisch eine Gegenüberstellung von Bestandteilen eines Lesemoduls in einem Analysesystem aus dem Stand der Technik und in einem erfindungsgemäßen Analysesystem,
- Figur 6: schematisch ein Lesemodul in einem erfindungsgemäßen Analysesystem,
- Figur 7: den Strahlengang in dem Lesemodul aus Figur 6,
- Figur 8: Platine und Mehrkomponentenspritzgießteil eines erfindungsgemäßen Analysesystems und
- Figur 9: Befestigungsvarianten für die Platine an dem Mehrkomponentenspritzgießteil.

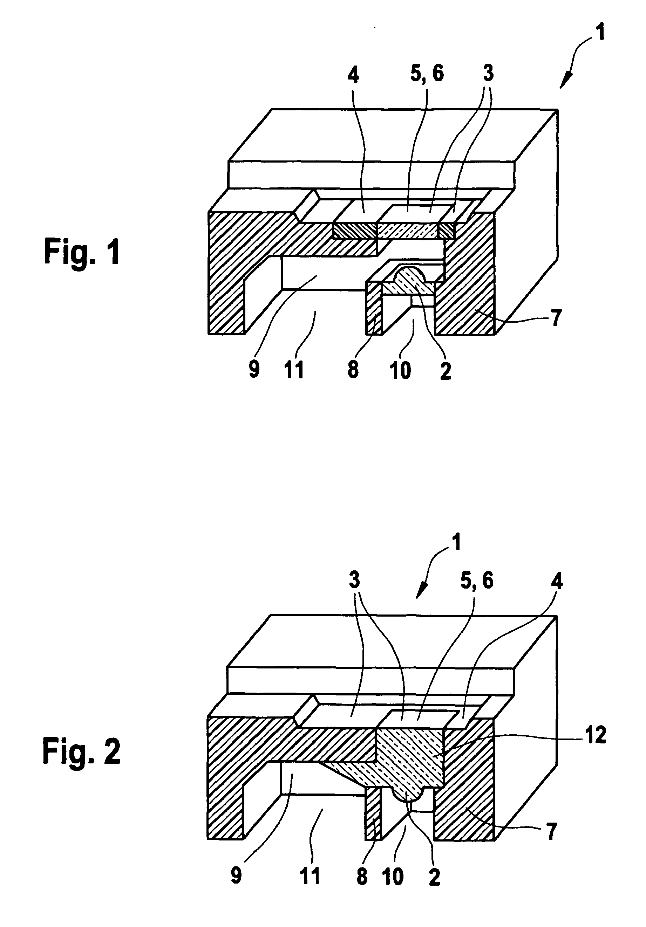

Figur 1 zeigt einen Schnitt durch ein Optikmodul, das in einem Messmodul eines Analysesystems aus dem Stand der Technik enthalten ist.

Das Optikmodul 1 umfasst eine Linse 2 und eine Blende 3, die einen Blendenkörper 4 und eine Blendenöffnung 5 enthält. Die Blendenöffnung 5 ist durch ein lichtdurchlässiges Fenster 6 ausgefüllt. Die Linse 2 und die Blende 3 sind einzelne Bauteile, die mit einem Optikmodulgrundkörper 7 verklebt sind. Die Einfassung 8 der Linse 2 dient als eine weitere Blende. In dem Optikmodulgrundkörper 7 befinden sich ein großer Hohlraum 9 und ein kleiner Hohlraum 10. Lichtstrahlen von einer (nicht dargestellten) Lichtquelle können aus dem kleinen Hohlraum 10 durch die Linse 2, durch den großen Hohlraum 9 und durch das die Blendenöffnung 5 verschließende lichtdurchlässige Fenster 6 zu einem (nicht dargestellten) Testelement gelangen, das sich über der Blende 3 in einer Testelementaufnahme 15 befindet. An dem Testelement reflektiertes Licht kann dann zurück durch das Fenster 6 in den großen Hohlraum 9 gelangen und von dort aus durch eine Öffnung 11 zu einem (nicht dargestellten) Detektor. Dieses Optikmodul aus vielen miteinander verklebten Einzelteilen weist die bereits genannten Nachteile des Standes der Technik auf.

Figur 2 zeigt einen Schnitt durch ein Optikmodul, das in einem Messmodul eines erfindungsgemäßen Analysesystems enthalten ist.

Das Optikmodul 1 ist in diesem Fall ein Zweikomponentenspritzgießteil, das die Blende 3 (incl. Blendenkörper 4 und als Blendenöffnung 5 dienenden Fenster 6), die Linse 2 und den Optikmodulgrundkörper 7 einstückig zusammenfasst. Der Optikmodulgrundkörper 7, der Blendenkörper 4 und die Einfassung 8 der Linse 2 sind dabei aus einem lichtundurchlässigen Kunststoff gespritzt. Die Linse 2 und das Fenster 6 in der Blendenöffnung 5 sind in einem durchgehenden Bereich 12 aus einem lichtdurchlässigen Kunststoff in dem Zweikomponentenspritzgießteil zusammengefasst.

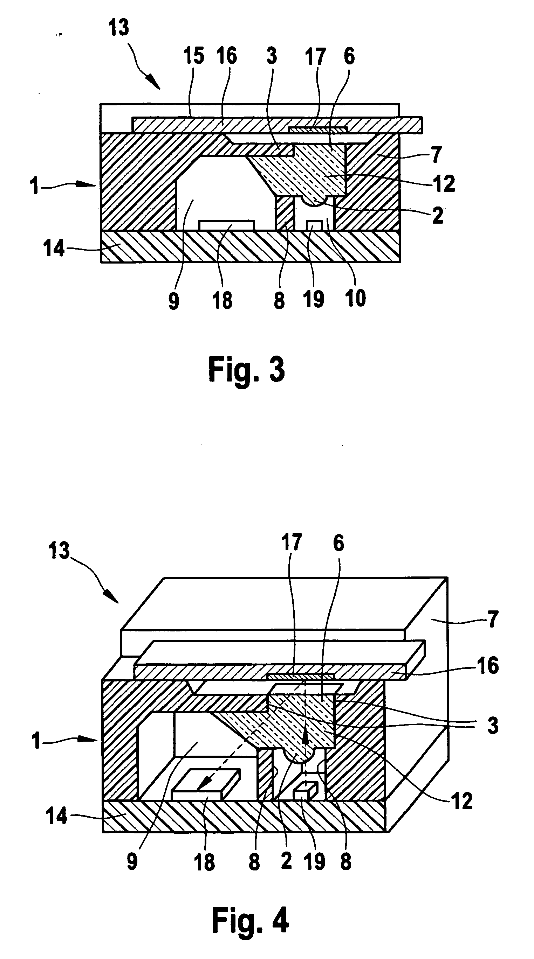

Figur 3 zeigt einen Schnitt durch ein Messmodul eines erfindungsgemäßen Analysesystems mit Testelement.

In dem Messmodul 13 sind ein Optikmodul 1 gemäß Figur 2 und eine Platine 14 enthalten. Das Optikmodul 1 umfasst den Optikmodulgrundkörper 7, die Linse 2, die Blende 3, die Einfassung 8 der Linse 2, die Hohlräume 9, 10 und eine Testelementaufnahme 15. Das Optikmodul 1 ist als Zweikomponentenspritzgießteil mit einer ersten, lichtdurchlässigen Kunststoffkomponente (Bereich 12) und einer zweiten, lichtundurchlässigen Kunststoffkomponente ausgeführt. In der Testelementaufnahme 15 befindet sich ein streifenförmiges analytisches Testelement 16, dessen Testfeld 17, in dem sich eine zu analysierende Probe befindet, oberhalb des Fensters 6 angeordnet ist.

Auf der Platine 14 befinden sich ein Detektor 18 und eine Lichtquelle 19, die in den großen Hohlraum 9 beziehungsweise in den kleinen Hohlraum 10 hineinragen. Die Platine 14 ist beispielsweise an dem Zweikomponentenspritzgießteil festgeklebt.

Figur 4 zeigt den Strahlengang in dem Messmodul gemäß Figur 3.

Die Lichtquelle 19 emittiert Licht zur photometrischen Analyse einer Probe auf dem Testfeld 17 des Testelements 16, das durch den durchgehenden lichtdurchlässigen Bereich 12 auf das Testelement 17 fokussiert wird. An dem Testfeld 17 wird in Abhängigkeit von dessen optischen Eigenschaften (zum Beispiel dessen Färbung) ein Teil des Lichts reflektiert und fällt durch den Bereich 12 zurück in den großen Hohlraum 9 auf den Detektor 18, aus dessen Signal die Konzentration eines Analyten in der Probe bestimmt werden kann (zum Beispiel in einem nicht dargestellten Auswertemodul des erfindungsgemäßen Analysesystems).

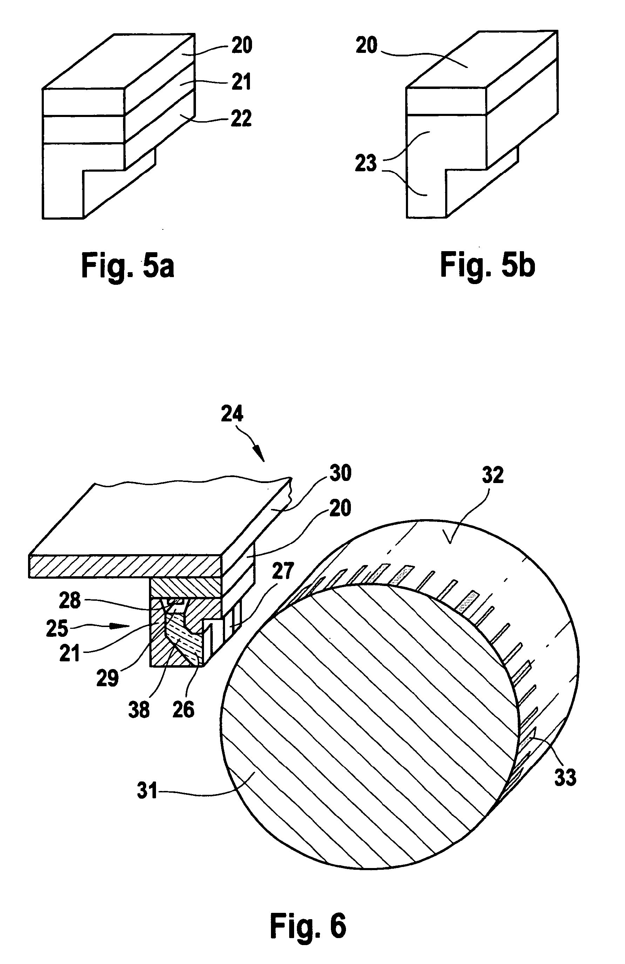

Figur 5 zeigt schematisch eine Gegenüberstellung von Bestandteilen eines Lesemoduls in einem Analysegerät aus dem Stand der Technik und in einem erfindungsgemäßen Analysesystem.

Im Stand der Technik (Figur 5a) umfasst ein Lesemodul eine Platine 20, eine Blende 21 und eine Linsenanordnung 22 mit mindestens einer Linse, wobei diese als drei getrennte Bauteile hergestellt und nachträglich zusammengefügt werden.

Gemäß der vorliegenden Erfindung (Figur 5b) sind in dem Lesemodul die Linsenanordnung 22 und die Blende 21 in einem Mehrkomponentenspritzgießteil 23 einstückig zusammengefasst. Das Mehrkomponentenspritzgießteil ist mit der Platine 20 zusammengefügt.

Figur 6 zeigt schematisch ein Lesemodul in einem erfindungsgemäßen Analysesystem.

Das Lesemodul 24 enthält unter anderem ein Optikmodul 25 und eine Platine 20. In dem Optikmodul 25 sind eine erste Linse 26 (im Schnitt gezeigt), eine zweite Linse 27 und eine Blende 21 zu einem einstückigen Zweikomponentenspritzgießteil zusammengefasst. Die Linsen 26, 27 sind aus einer ersten, lichtdurchlässigen Komponente (lichtdurchlässiger Bereich 38) und die Blende 21 aus einer zweiten, lichtundurchlässigen Komponente gespritzt. Die Platine 20 ist mit dem Optikmodul zum Beispiel über eine Klebeverbindung verbunden. Sie trägt eine Lichtquelle 28 und einen (nicht dargestellten) Detektor, die in Hohlräume 29 des Optikmoduls 25 hineinragen. Die Platine ist zum Beispiel an einer Analysesystemplatine 30 befestigt.

In Figur 6 ist ferner ein trommelförmiges Testelement-Magazin 31 dargestellt, das zur Bevorratung einer Vielzahl von Testelementen dient. An seiner Mantelfläche 32 weist das Magazin 31 einen Barcode 33 auf, den das Lesemodul 24 lesen kann. Das Magazin 31 befindet sich in einer Magazinaufnahme in dem Lesemodul 24, wodurch es relativ zu dem Optikmodul 25 zum Lesen des Barcodes 33 positioniert wird.

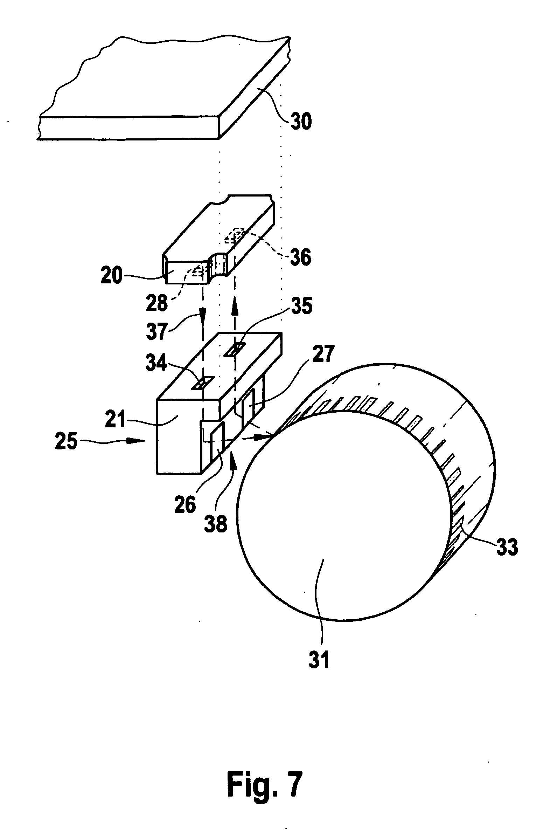

Figur 7 zeigt den Strahlengang in dem Lesemodul aus Figur 6.

Zur besseren Anschaulichkeit sind die Analysesystemplatine 30, die Platine 20 und das Optikmodul 25 getrennt dargestellt. Der Strahlengang 37 wird durch Pfeile angedeutet. Licht von einer Lichtquelle 28 breitet sich durch den ersten Hohlraum 34, die Blende 21 und dem lichtdurchlässigen Bereich 38 incl. der ersten Linse 26 zu dem Barcode 33 hin aus, wird daran reflektiert und erreicht durch den lichtdurchlässigen Bereich 38 mit der zweiten Linse 27, die Blende 21 und den zweiten Hohlraum 35 den Detektor 36.

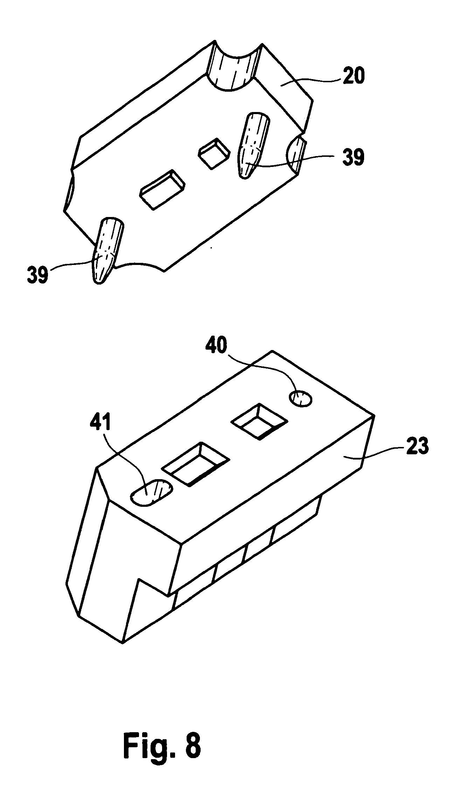

Figur 8 demonstriert, wie die Platine mit dem Mehrkomponentenspritzgießteil in einem erfindungsgemäßen Analysesystem miteinander verbunden werden kann.

Dazu weist die Platine 20 zwei Stifte 39, die in dafür vorgesehene Öffnungen (Bohrung 40 in Langloch 41) in dem Mehrkomponentenspritzgießteil 23 eingreifen können. Dabei werden die Lichtquelle 28 und der Detektor 36 so positioniert, dass sie exakt innerhalb der Hohlräume 34, 35 ausgerichtet sind. Dann werden die beiden Teile 20, 23 durch ein dem Fachmann bekanntes Fügeverfahren zusammengefügt. Das Mehrkomponentenspritzgießteil 23 ist zum Beispiel das Optikmodul eines Lesemoduls.

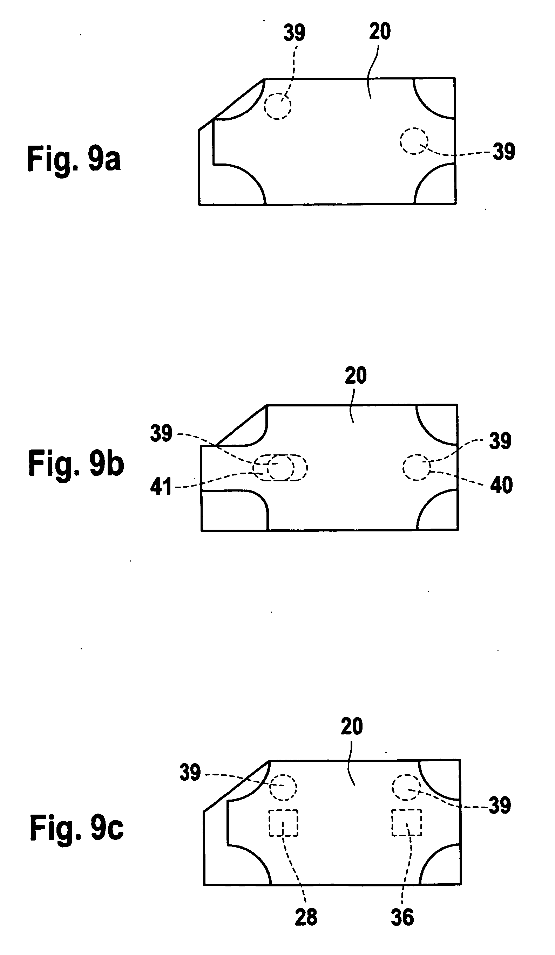

Figur 9 zeigt verschiedene Befestigungsvarianten a) bis c) für die Platine.

In Variante a) weist die Platine 20 zwei Stifte 39 auf, die zueinander versetzt angeordnet sind. Variante b) entspricht der in Figur 8 gezeigten Variante mit zwei auf einer mittleren Linie liegenden Stiften 39, die in eine Bohrung 40 und in ein gefrästes Langloch 41 eingreifen. In Variante c) liegen die zwei Stifte 39 auf einer gemeinsamen seitlichen Linie und auf einer Linie in Verlängerung zu der Lichtquelle 28 beziehungsweise zu dem Detektor 36.

### Bezugszeichenliste

- 1: Optikmodul
- 2: Linse
- 3: Blende
- 4: Blendenkörper
- 5: Blendenöffnung
- 6: lichtdurchlässiges Fenster
- 7: Optikmodulgrundkörper
- 8: Einfassung der Linse
- 9: großer Hohlraum
- 10: kleiner Hohlraum
- 11: Öffnung
- 12: durchgehender Bereich
- 13: Messmodul
- 14: Platine
- 15: Testelementaufnahme
- 16: Testelement
- 17: Testfeld
- 18: Detektor
- 19: Lichtquelle
- 20: Platine
- 21: Blende
- 22: Linsenanordnung
- 23: Mehrkomponentenspritzgießteil
- 24: Lesemodul
- 25: Optikmodul
- 26: erste Linse
- 27: zweite Linse
- 28: Lichtquelle
- 29: Hohlräume
- 30: Analysesystem Platine
- 31: Testelement-Magazin
- 32: Mantelfläche
- 33: Barcode
- 34: erster Hohlraum
- 35: zweiter Hohlraum
- 36: Detektor
- 37: Strahlengang
- 38: lichtdurchlässiger Bereich
- 39: Stifte
- 40: Bohrung
- 41: Langloch

## Patentansprüche

1. Analysesystem zur Analyse einer Probe auf einem analytischen Testelement (16), umfassend
• ein Messmodul (13) zur Durchführung von Messungen an der Probe auf dem analytischen Testelement (16) und
• ein Optikmodul (1, 25), das eine Linse (2, 26, 27) und eine Blende (3, 21) umfasst, durch die Licht fokussiert werden kann,
**dadurch gekennzeichnet, dass** die Linse (2, 26, 27) und die Blende (3, 21) des Optikmoduls (1, 25) einstückig in einem Mehrkomponentenspritzgießteil (23) zusammengefasst sind.

2. Analysesystem gemäß Anspruch 1, **dadurch gekennzeichnet, dass** das Mehrkomponentenspritzgießteil (23) ein Zweikomponentenspritzgießteil mit einer ersten lichtdurchlässigen Kunststoffkomponente und einer zweiten lichtundurchlässigen Kunststoffkomponente ist.

3. Analysesystem gemäß einem der Ansprüche 1 oder 2, **dadurch gekennzeichnet, dass** die Blende (3, 21) einen Blendenkörper (4) aus einem lichtundurchlässigen Kunststoff und eine Blendenöffnung (5) umfasst, wobei die Blendenöffnung (5) mit einem lichtdurchlässigen Kunststoff gefüllt ist.

4. Analysesystem gemäß Anspruch 3, **dadurch gekennzeichnet, dass** die Linse (2, 26, 27) aus dem lichtdurchlässigen Kunststoff besteht.

5. Analysesystem gemäß Anspruch 4, **dadurch gekennzeichnet, dass** die Linse (2, 26, 27) und die mit dem lichtdurchlässigen Kunststoff gefüllte Blendenöffnung (5) in einem durchgehenden Bereich (12) aus dem lichtdurchlässigen Kunststoff in dem Mehrkomponentenspritzgießteil (23) zusammengefasst sind.

6. Analysesystem gemäß einem der Ansprüche 1 oder 2, **dadurch gekennzeichnet, dass** die Blende (3, 21) einen Blendenkörper (4) aus einem lichtundurchlässigen Kunststoff und eine Blendenöffnung (5) umfasst, wobei die Blendenöffnung (5) eine Aussparung in dem Blendenkörper (4) ist.

7. Analysesystem gemäß einem der Ansprüche 1 bis 6, **dadurch gekennzeichnet, dass** das Messmodul (13) das Optikmodul (7) enthält, wobei das Optikmodul (7) zur Durchführung von optischen Messungen an der Probe auf dem analytischen Testelement (16) dient.

8. Analysesystem gemäß Anspruch 7, **dadurch gekennzeichnet, dass** das Messmodul (13) eine Lichtquelle (19), einen Detektor (18) und eine Testelementaufnahme (15) umfasst, die so angeordnet sind, dass Licht von der Lichtquelle (19) durch einen lichtdurchlässigen Bereich (12) des Mehrkomponentenspritzgießteils (23) zu einem in der Testelementaufnahme (15) angeordneten Testelement (16) und reflektiert von dem Testelement (16) durch den lichtdurchlässigen Bereich (12) zu dem Detektor (18) gelangen kann.

9. Analysesystem gemäß einem der Ansprüche 1 bis 8, **gekennzeichnet durch** ein Lesemodul (24) zum Lesen optisch codierter Daten, wobei das Lesemodul (24) das oder ein weiteres Optikmodul (25) enthält.

10. Analysesystem gemäß Anspruch 9, **dadurch gekennzeichnet, dass** das Lesemodul (24) ferner eine Lichtquelle (28), einen Detektor (36) und eine Magazinaufnahme umfasst, die so angeordnet sind, dass Licht von der Lichtquelle (28) durch einen lichtdurchlässigen Bereich (38) des Mehrkomponentenspritzgießteils (23) zu einem in der Magazinaufnahme aufgenommenen Testelement-Magazin (31) und reflektiert von dem Testelement-Magazin (31) durch den lichtdurchlässigen Bereich (38) zu dem Detektor (36) gelangen kann.

11. Analysesystem gemäß Anspruch 10, **dadurch gekennzeichnet, dass** die Lichtquelle (28) und der Detektor (36) auf einer Platine (20) angeordnet sind, die mit dem Optikmodul (25) verbunden ist.

12. Verfahren zur Herstellung eines Analysesystems zur Analyse einer Probe auf einem analytischen Testelement (16), wobei das Analysesystem ein Messmodul (13) und ein Optikmodul (1, 25) enthält, wobei das Optikmodul eine Linse (2, 26, 27) und eine Blende (3, 21) umfasst, **gekennzeichnet durch** die Schritte:
• Mehrkomponentenspritzgießen der Linse und der Blende in ein einstückiges Mehrkomponentenspritzgießteil und
• Positionieren und Montieren des Mehrkomponentenspritzgießteils in dem Analysesystem.

## Claims

1. An analysis system for analysing a sample on an analytical test element (16), including
• a measuring module (13) for performing measurements in respect of the sample on the analytical test element (16) and
• an optical module (1, 25) which includes a lens (2, 26, 27) and a diaphragm (3, 21) by which light can be focused,
**characterised in that** the lens (2, 26, 27) and the diaphragm (3, 21) of the optical module (1, 25) are integrally combined in a multi-component injection-moulded part (23).

2. Analysis system according to Claim 1, **characterised in that** the multi-component injection-moulded part (23) is a two-component injection-moulded part with a first, translucent synthetic component and with a second, opaque synthetic component.

3. Analysis system according to one of Claims 1 or 2, **characterised in that** the diaphragm (3, 21) includes a diaphragm body (4), consisting of a opaque synthetic material, and a diaphragm aperture (5), said diaphragm aperture (5) being filled with a translucent synthetic material.

4. Analysis system according to Claim 3, **characterised in that** the lens (2, 26, 27) consists of the translucent synthetic material.

5. Analysis system according to Claim 4, **characterised in that** the lens (2, 26, 27) and the diaphragm aperture (5) filled with the translucent synthetic material are combined in an uninterrupted region (12) consisting of the translucent synthetic material in the multi-component injection-moulded part (23).

6. Analysis system according to one of Claims 1 or 2, **characterised in that** the diaphragm (3, 21) includes a diaphragm body (4), consisting of a translucent synthetic material, and a diaphragm aperture (5), said diaphragm aperture (5) being a recess in the diaphragm body (4).

7. Analysis system according to one of Claims 1 to 6, **characterised in that** the measuring module (13) contains the optical module (7), said optical module (7) serving for performing optical measurements in respect of the sample on the analytical test element (16).

8. Analysis system according to Claim 7, **characterised in that** the measuring module (13) includes a light-source (19), a detector (18) and a test-element receptacle (15) which are arranged in such a way that light from the light-source (19) is able to pass through a translucent region (12) of the multi-component injection-moulded part (23) to a test element (16) arranged in the test-element receptacle (15) and, reflected from the test element (16), through the translucent region (12) to the detector (18).

9. Analysis system according to one of Claims 1 to 8, **characterised by** a read module (24) for reading optically coded data, said read module (24) containing the optical module (25) or a further optical module.

10. Analysis system according to Claim 9, **characterised in that** the read module (24) further includes a light-source (28), a detector (36) and a magazine receptacle which are arranged in such a way that light from the light-source (28) is able to pass through a translucent region (38) of the multi-component injection-moulded part (23) to a test-element magazine (31) received in the magazine receptacle and, reflected from the test-element magazine (31), through the translucent region (38) to the detector (36).

11. Analysis system according to Claim 10, **characterised in that** the light-source (28) and the detector (36) are arranged on a plate (20) which is connected to the optical module (25).

12. A process for producing an analysis system for analysing a sample on an analytical test element (16), said analysis system containing a measuring module (13) and an optical module (1, 25), said optical module including a lens (2, 26, 27) and a diaphragm (3, 21), **characterised by** the following steps:
• multi-component injection moulding of the lens and of the diaphragm into an integral multi-component injection-moulded part and
• positioning and mounting of the multi-component injection-moulded part in the analysis system.

## Revendications

1. Système d'analyse pour l'analyse d'un échantillon sur un élément de test (16) analytique comprenant
• un module de mesure (13) pour la réalisation de mesures sur l'échantillon sur l'élément de test (16) analytique et
• un module d'optique (1, 25), qui comporte une lentille (2, 26, 27) et un diaphragme (3, 21), par lequel de la lumière peut être focalisée,
**caractérisé en ce que** la lentille (2, 26, 27) et le diaphragme (3, 21) du module d'optique (1, 25) sont regroupés d'un seul tenant dans une pièce moulée par injection à plusieurs composants (23).

2. Système d'analyse selon la revendication 1, **caractérisé en ce que** la pièce moulée par injection à plusieurs composants (23) est une pièce moulée par injection à deux composants comprenant un premier composant plastique perméable à la lumière et un second composant plastique imperméable à la lumière.

3. Système d'analyse selon l'une quelconque des revendications 1 ou 2, **caractérisé en ce que** le diaphragme (3, 21) comporte un corps de diaphragme (4) à base d'un plastique imperméable à la lumière et une ouverture de diaphragme (5), l'ouverture de diaphragme (5) étant remplie avec un plastique perméable à la lumière.

4. Système d'analyse selon la revendication 3, **caractérisé en ce que** la lentille (2, 26, 27) est constituée du plastique perméable à la lumière.

5. Système d'analyse selon la revendication 4, **caractérisé en ce que** la lentille (2, 26, 27) et l'ouverture de diaphragme (5) remplie avec le plastique perméable à la lumière sont regroupés dans une zone (12) continue à base du plastique perméable à la lumière dans la pièce moulée par injection à plusieurs composants (23).

6. Système d'analyse selon l'une quelconque des revendications 1 ou 2, **caractérisé en ce que** le diaphragme (3, 21) comprend un corps de diaphragme (4) à base d'un plastique imperméable à la lumière et une ouverture de diaphragme (5), l'ouverture de diaphragme (5) étant un évidement dans le corps de diaphragme (4).

7. Système d'analyse selon l'une quelconque des revendications 1 à 6, **caractérisé en ce que** le module de mesure (13) contient le module d'optique (7), le module d'optique (7) servant à la réalisation de mesures optiques sur l'échantillon sur l'élément de test (16) analytique.

8. Système d'analyse selon la revendication 7, **caractérisé en ce que** le module de mesure (13) comporte une source de lumière (19), un détecteur (18) et un logement d'élément de test (15), qui sont disposés de telle sorte que de la lumière peut arriver de la source lumineuse (19) à travers une zone (12) perméable à la lumière de la pièce moulée par injection à plusieurs composants (23) à un élément de test (16) disposé dans le logement d'élément de test (15) et, réfléchie par l'élément de test (16) à travers la zone (12) perméable à la lumière au détecteur (18).

9. Système d'analyse selon l'une quelconque des revendications 1 à 8, **caractérisé par** un module de lecture (24) pour la lecture de données codées visuellement, le module de lecture (24) contenant le ou un autre module d'optique (25).

10. Système d'analyse selon la revendication 9, **caractérisé en ce que** le module de lecture (24) comporte également une source lumineuse (28), un détecteur (36) et un logement de magasin, qui sont disposés de telle sorte que de la lumière peut arriver de la source lumineuse (28) à travers une zone (38) perméable à la lumière de la pièce moulée par injection à plusieurs composants (23) à un magasin d'élément de test (31) réceptionné dans le logement de magasin et, réfléchie par le magasin d'élément de test (31), à travers la zone (38) perméable à la lumière au détecteur (36).

11. Système d'analyse selon la revendication 10, **caractérisé en ce que** la source lumineuse (28) et le détecteur (36) sont disposés sur une platine (20) qui est reliée au module d'optique (25).

12. Procédé pour la fabrication d'un système d'analyse pour l'analyse d'un échantillon sur un élément de test (16) analytique, le système d'analyse contenant un module de mesure (13) et un module d'optique (1, 25), le module d'optique comportant une lentille (2, 26, 27) et un diaphragme (3, 21), **caractérisé par** les étapes suivantes :
• moulage par injection à plusieurs composants de la lentille et du diaphragme dans une pièce moulée par injection à plusieurs composants en un seul tenant et
• positionnement et montage de la pièce moulée par injection à plusieurs composants dans le système d'analyse.
